(19) Europäisches Patentamt
European Patent Office
Office européen des brevets

(11) Numéro de publication: **0 042 781**
**B1**

# (12) FASCICULE DE BREVET EUROPEEN

(45) Date de publication du fascicule du brevet: **06.03.85**

(21) Numéro de dépôt: **81400934.6**

(22) Date de dépôt: **12.06.81**

(51) Int. Cl.[4]: **C 07 D 401/06**, C 07 D 401/14, C 07 D 409/14, A 61 K 31/47 // C07D211/22, C07D211/34, C07D207/16

(54) **Nouveaux dérivés de (quinolyl-4)-1 (pipéridyl ou pyrrolidinyl-2 ou -3)-2 ou -3 éthanone ou propanone, procédés pour leur préparation, et leur utilisation comme médicaments.**

(30) Priorité: **20.06.80 FR 8013698**

(43) Date de publication de la demande: **30.12.81 Bulletin 81/52**

(45) Mention de la délivrance du brevet: **06.03.85 Bulletin 85/10**

(84) Etats contractants désignés: **BE CH DE FR GB IT LI LU NL SE**

(56) Documents cités:
**FR - A - 2 206 944**
**FR - A - 2 354 771**
**FR - A - 2 381 044**
**US - A - 2 492 487**

**E. SCHRÖDER et al., Arzneimittelchemie II, Georg Thieme Verlag, Stuttgart, page 84, 1976**
**KIRK-OTHMER, Encyclopedia of Chemical Technology, Vol. 8 1952, page 673**

**Le dossier contient des informations techniques présentées postérieurement au dépôt de la demande et ne figurant pas dans le présent fascicule.**

(73) Titulaire: **PHARMUKA LABORATOIRES, 35 Quai du Moulin de Cage, F-92231 Gennevilliers (FR)**

(72) Inventeur: **Dubroeucq, Marie-Christine, 13, Villa Malleville, F-95880 Enghien-les-Bains (FR)**
Inventeur: **Gueremy, Claude Georges Alexandre, 3, rue Daumesnil, F-78800 Houilles (FR)**
Inventeur: **Le Fur, Gérard Roger, 35, rue du Progrès, F-92350 Plessis-Robinson (FR)**
Inventeur: **Mizoule, Jacques, Résidence Sisley 3, avenue de Verdun, F-92390 Villeneuve-la-Garenne (FR)**

(74) Mandataire: **Gaumont, Robert et al, RHONE-POULENC RECHERCHES Service Brevets Pharma 25, Quai Paul Doumer, F-92408 Courbevoie Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 042 781 B1

## Description

La présente invention a pour objet de nouveaux dérivés de (quinolyl-4)-1 pipéridyl ou pyrrolidinyl-2 ou -3)-2 ou -3 éthanone ou propanone utilisables comme médicaments ou comme produits intermédiaires pour la préparation de médicaments.

Ces dérivés peuvent être représentés par la formule générale:

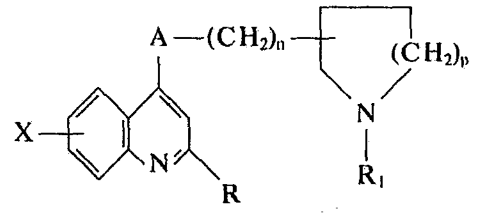

(I)

dans laquelle

R représente un groupe phényle, pyridyle ou un groupe phényle substitué par un ou deux substituants pris parmi les atomes d'halogène (chlore, fluor, brome) et le groupe trifluorométhyle,

X est fixé en position 6 sur le cycle de la quinoléine et représente un atome d'hydrogène ou d'halogène (chlore, fluor, brome), ou un groupe méthoxy,

$R_1$ représente un atome d'hydrogène, un groupe alkyle comportant de 1 à 4 atomes de carbone,

A représente un groupe CO, ou $CH_2$,

n est un nombre entier égal à 1 ou 2, p est un nombre entier égal à 1 ou 2, et le groupe

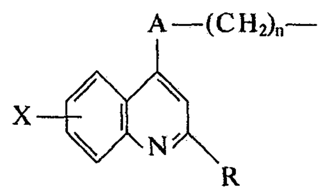

est fixé en position 2 ou 3 sur l'hétérocycle azoté

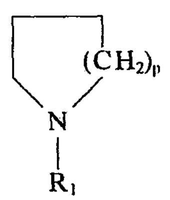

Comme l'indique la formule (I) ci-dessus, les composés selon l'invention comportent un cycle pyrrolidinique (cas p = 1) ou un cycle pipéridinique (cas p = 2).

Lorsque A est un groupe CO ou $CH_2$, la molécule des composés de formule (I) comporte un atome de carbone asymétrique et donc, pour une signification donnée de R, X, A, $R_1$, n et p, il y a un racémique et deux énantiomères correspondant à la formule plane (I).

Dans la formule (I) des composés selon l'invention, X est de préférence un atome d'hydrogène, R un groupe phényle et n est de préférence égal à 1.

Ces composés sont utiles comme antiarythmiques, hypnotiques, anticonvulsivants et pour le traitement des états d'anxiété et de divers états psychonevrotiques.

Des dérivés de [(quinolyl-4)-3 propyl-1]-4 piperidine sont décrits dans le brevet FR 2 354 771 comme présentant une activité psychotrope et antidépressive.

Les composés de formule (I) pour lesquels A représente le groupe $CH_2$ peuvent être préparés par réduction des composés correspondants de formule (I) pour lesquels A représente le groupe CO.

Pour cette réduction on utilise des méthodes, connues en soi, qui permettent de transformer un groupement CO en groupement $CH_2$, par exemple celles décrites par R.B. Wagner et H. D. Zook (Synthetic Organic Chemistry, p. 5, J. Wiley and Sons, 1953). On utilise avantageusement comme agent réducteur l'hydrate d'hydrazine en présence d'un hydroxyde de métal alcalin tel que l'hydroxyde de sodium ou de potassium, au sein d'un solvant inerte tel qu'un alcool ou un diol, par exemple le diéthylèneglycol ou l'éthylèneglycol, à une température comprise entre 100 et 180°C.

Les composés de formule (I) pour lesquels A représente le groupe CO et $R_1$ l'atome d'hydrogène peuvent être préparés par condensation d'un ester d'acide quinoléine-carboxylique de formule (II) avec un ester de formule (III), puis hydrolyse et décarboxylation du composé de formule (IV) ainsi obtenu, selon le schéma réactionnel suivant:

a)

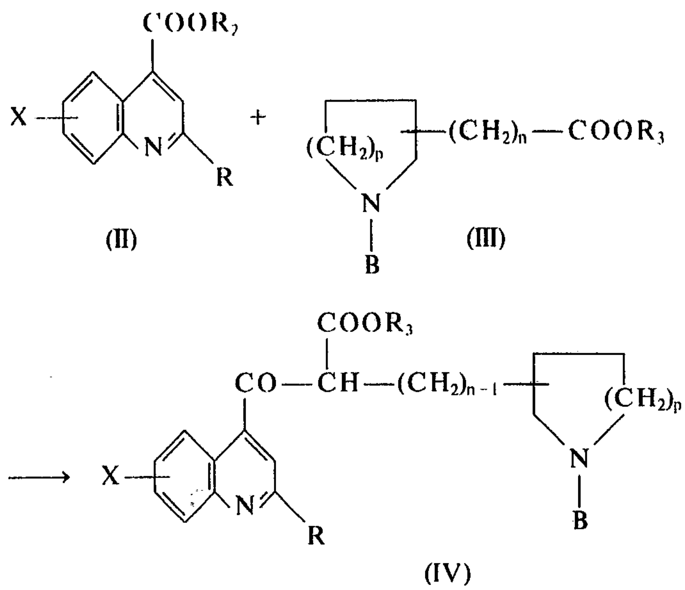

(II) (III)

+ $R_2OH$

(IV)

b)

(IV) + 2 $H_2O$ ⟶

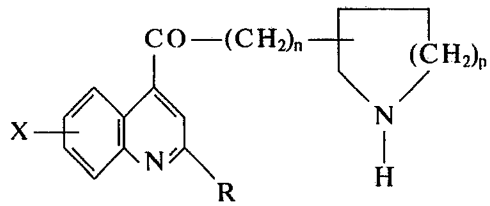

+ $CO_2$ + $R_3OH$ + BOH

Dans les formules (II), (III), et (IV) ci-dessus, X, R, n et p ont la même signification que dans la formule (I), $R_2$ et $R_3$ représentent des groupes alkyle de bas poids moléculaire, par exemple méthyle ou éthyle, et B représente un groupe protecteur de la fonction amine, stable en milieu alcalin anhydre et susceptible d'être éliminé en milieu acide, tels que ceux qui sont décrits par R. A. Boissonnas, Advances in Organic Chemistry 3, p. 159, Interscience (1963). On utilise avantageusement comme groupe B un groupe acyle tel que le groupe benzoyle, le groupe benzyloxycarbonyl ou le groupe triphénylméthyle.

Pour réaliser la réaction de condensation a) on fait appel à des procédés connus en soi (cf. »The acetoacetic acid ester condensation«, C. R. Hauser et al., Organic Reactions, vol. 1, p. 266, Wiley and Sons, 1942). On opère avantageusement en présence d'une base telle qu'un alcoolate (par exemple le tertiobutylate de potassium) ou un hydrure métallique (par exemple l'hydrure de sodium ou de potassium), au sein d'un solvant inerte tel qu'un hydrocarbure ou un autre solvant aprotique (par exemple le tétrahydrofuranne), à une température comprise entre 0°C et la température d'ébullition du solvant utilisé.

La réaction d'hydrolyse b) est conduite selon des procédés connus en soi (cf. »Cleavage of $\beta$-ketoesters«, R. B. Wagner et H. D. Zook, Synthetic Organic Chemistry, P. 327, Wiley and Sons, 1953). La méthode la plus courante consiste à chauffer à l'ébullition le produit de formule (IV) dans une solution aqueuse d'un acide tel que l'acide chlorhydrique ou l'acide sulfurique.

Les produits de départ de formule (II) sont facilement accessibles par des méthodes décrites dans la littérature (cf. Quinolines-Heterocyclic compounds — 32, 274, Wiley and Sons, 1977). Les produits de formule (III) peuvent être préparés par exemple par benzoylation des esters de formule:

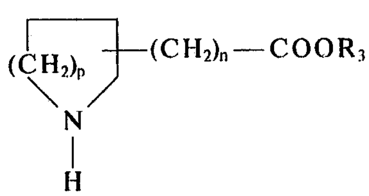

(V)

Dans la réaction de benzoylation, on utilise avantageusement le chlorure de benzoyle, généralement en présence d'une base telle que la triéthylamine ou un carbonate de métal alcalin, au sein d'un solvant inerte tel que le chloroforme, à une température comprise entre 0°C et 50°C.

Les esters de formule (V) pour lesquels $R_3$ est le groupe éthyle sont des produits connus. Les autres esters de formule (V) peuvent être préparés par des procédés connus décrits dans la littérature.

Les composés de formule générale (I) pour lesquels $R_1$ est un groupe alkyle, peuvent être préparés par action sur les composés correspondants de formule (I) pour lesquels $R_1$ est un atome d'hydrogène d'un

agent alkylant. Comme agents alkylant on peut citer en particulier les halogénures de formule $R_1Hal$, les sulfates de formule $(R_1)_2\ SO_4$ et les alkyl- ou arylsulfonates de formule $ArSO_3R_1$ ou $R'SO_3R_1$, formules dans lesquelles $R_1$ représente un groupe alkyle ayant 1 à 4 atomes de carbone, Ar représente un groupe aryle et R' représente un groupe alkyle.

La réaction d'alkylation des composés de formule (I) pour lesquels $R_1 = H$ au moyen de l'agent alkylant est effectuée selon des procédés connus en soi. On opère avantageusement en présence d'une base organique ou minérale (par exemple le carbonate de sodium ou de potassium), au sein d'un solvant inerte, par exemple le diméthylformamide.

Les composés de formule (I) pour lesquels $R_1$ représente le groupe méthyle et A le groupe $CH_2$ peuvent également être préparés par action sur les composés correspondants de formule (I) pour lesquels $R_1$ est un atome d'hydrogène du formol au sein de l'acide formique (réaction de Leuckart: cf. Organic Reactions, Vol. 5, p. 301, John Wiley and Sons, 1949). Cette réaction est effectuée avantageusement à une température voisine de 100°C.

Une variante pour la préparation des produits de formule générale (I) pour lesquels A représente le groupe $CH_2$ et $R_1$ un groupe alkyle comportant 2 à 4 atomes de carbone ou arylalkyle consiste à faire réagir sur les produits de formule (I) correspondants pour lesquels A représente le groupe $CH_2$ et $R_1$ l'atome d'hydrogène un chlorure d'acide de formule

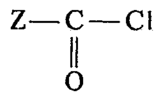

dans laquelle Z représente un groupe alkyle ayant 1 à 3 atomes de carbone, un groupe aryle ou un groupe arylalkyle dont la partie alkyle a un ou deux atomes de carbone, et à réduire ensuite au moyen d'un hydrure le composé de formule (VI) ainsi obtenu, selon le schéma réactionnel suivant:

c)

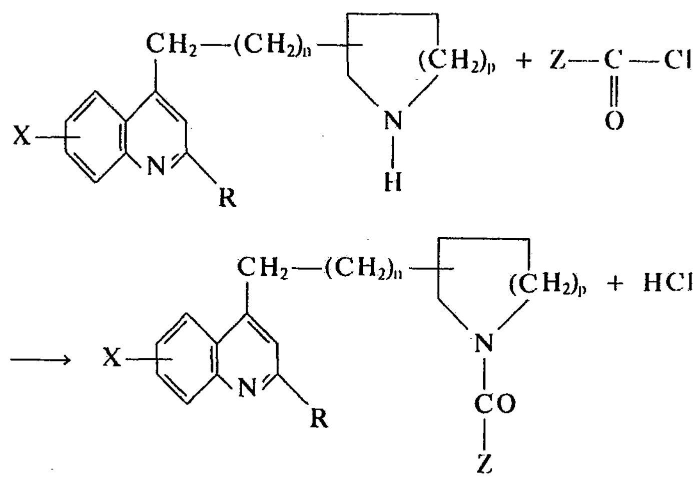

(VI)

d)

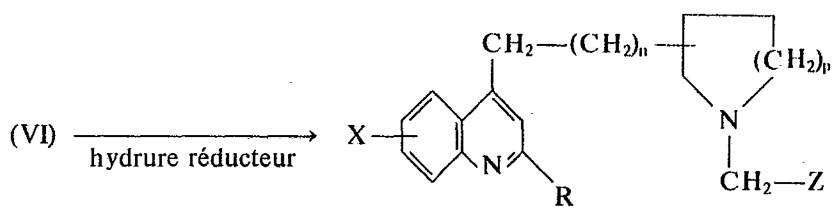


Pour effectuer la réaction c) on utilise des méthodes, connues en soi, qui permettent de transformer une amine secondaire en amide, par exemple celles décrites par R. B. Wagner et. H. D. Zook (Synthetic Organic Chemistry, p. 565 et p. 646, Wiley and Sons, 1953). On opère généralement en présence d'une base telle que la triéthylamine, au sein d'un solvant inerte tel que le chloroforme à une température comprise entre 0°C et 50°C.

La réaction d) utilise également des méthodes connues en soi. On utilise avantageusement comme hydrure réducteur l'hydrure d'aluminium et de lithium, au sein d'un solvant inerte tel qu'un éther (par exemple oxyde diéthylique, tétrahydrofuranne) ou un hydrocarbure aromatique, à une température comprise entre 0°C et la température d'ébullition du solvant utilisé.

Les produits de formule (I) répondant à la formule:

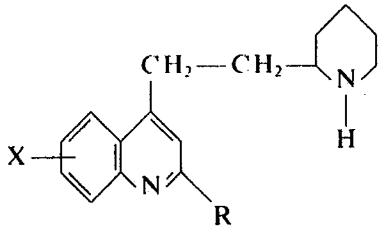

(VII)

peuvent être également préparés par hydrogénation catalytique des dérivés pyridiniques de formule:

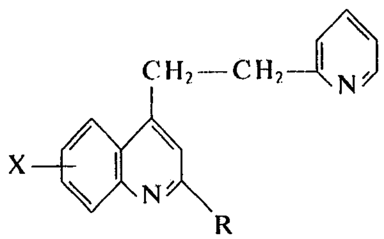

(VIII)

Pour effectuer cette hydrogénation, on opère avantageusement à une température comprise entre 20°C et 60°C, sous une pression d'hydrogène voisine de 1 bar, en présence d'oxyde de platine comme catalyseur et au sein d'un solvant inerte tel que l'acide acétique ou un mélange d'un alcool (en particulier le méthanol ou l'éthanol) et d'un acide (en particulier l'acide acétique ou l'acide chlorhydrique).

Les composés de formule (VIII) peuvent être préparés par réaction de la méthyl-2 pyridine avec un dérivé métallique de formule R''M, dans laquelle M désigne un métal alcalin (en particulier le lithium, le sodium, le potassium) et R'' désigne un atome d'hydrogène, un groupe $NH_2$, amino disubstitué, alkyle ou aryle, réaction de la méthyl-2 pyridine métallée de formule (IX) ainsi obtenue avec un ester d'acide quinoléinecarboxylique de formule (II) et réduction du dérivé carbonylé de formule (X) ainsi abtenu. L'ensemble des réactions peut être schématisé comme suit:

e) 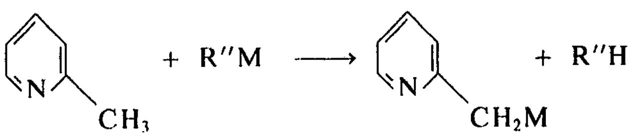

f) (IX) + (II) 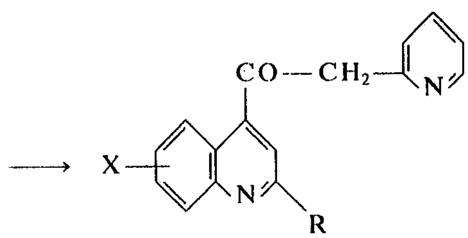

(X)

g) (X) $\xrightarrow{\text{réduction}}$ (VIII)

Les réactions e) et f) sont effectuées au sein d'un solvant inerte tel qu'un éther (par exemple l'oxyde de diéthyle, le tétrahydrofuranne ou le diméthoxyéthane), de préférence à une température comprise entre −60°C et −70°C. Comme dérivé métallique R''M on utilise de préférence un dérivé du lithium, par exemple le phényllithium, le butyllithium ou le diisopropylamidure de lithium.

Pour la réaction g) on utilise avantageusement comme agent réducteur l'hydrate d'hydrazine en présence d'un hydroxyde de métal alcalin tel que l'hydroxyde de sodium ou de potassium, au sein d'un solvant inerte tel qu'un alcool ou un diol, par exemple le diéthylèneglycol, à une température comprise entre 100 et 180°C.

Les composés de formule (I) répondant à la formule:

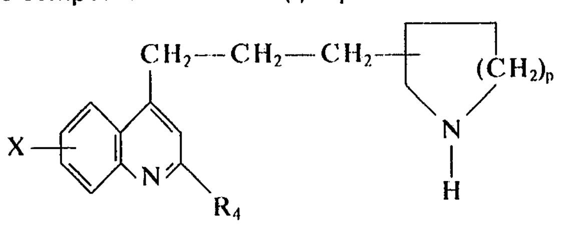

(XI)

dans laquelle X et p ont la même signification que dans la formule (I) et $R_4$ désigne un atome d'hydrogène, un groupe phényle ou un groupe phényle substitué par un ou deux substituants pris parmi les

atomes d'halogène, les groupes alkyle, alcoxy et alkylthio ayant 1 à 4 atomes de carbone et $CF_3$, peuvent également être préparés par réaction d'un dérivé métallique R"M tel que défini précédemment avec un dérivé de la quinoléine de formule (XII), réaction du composé de formule (XIII) ainsi obtenu avec un composé de formule:

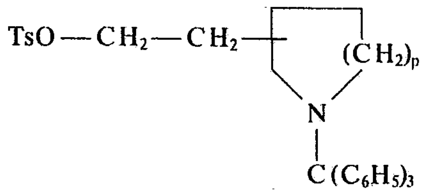

(XIV)

dans laquelle Ts désigne le groupe tosyle, c'est-à-dire le groupe

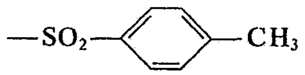

et action d'un acide sur le composé de formule (XV) ainsi obtenu. L'ensemble des réactions peut être schématisé comme suit:

h) 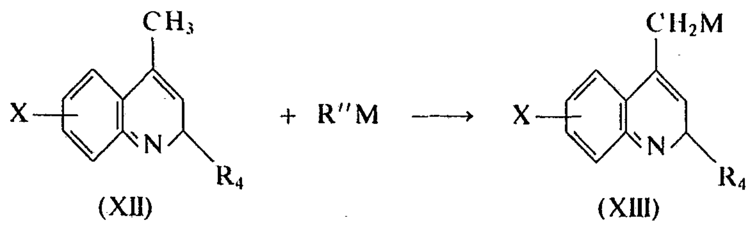


(XII) (XIII)

(XIV)

i) (XIII) + (XIV) ⟶ 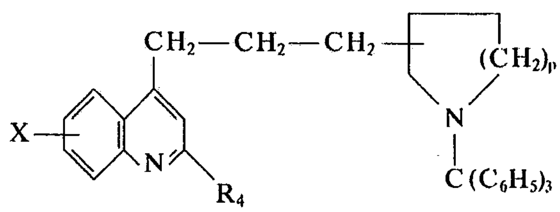

(XV)

j) (XV) $\xrightarrow{\text{acide}}$ (XI)

Les réactions h) et i) sont effectuées au sein d'un solvant inerte tel qu'un éther (par exemple l'oxyde de diéthyle, le tétrahydrofuranne ou le diméthoxyéthane), à une température qui est fonction du dérivé métallique R"M utilisé et qui peut aller de $-70°C$ jusqu'à la température d'ébullition du solvant utilisé. Comme dérivé métallique R"M on utilise de préférence un dérivé du lithium, par exemple le phényllithium, le butyllithium ou le diisopropylamidure de lithium, auquel cas la réaction h) est effectuée à une température de $-70°C$ à $0°C$ et la réaction i) à une température comprise entre $-70°C$ et la température ambiante.

La réaction j), qui consiste à remplacer le groupe protecteur triphénylméthyle par un atome d'hydrogène, est effectuée en traitant le composé de formule (XV) par un acide tel que l'acide chlorhydrique, en milieu hydroalcoolique, à une température comprise entre 20°C et 70°C.

Les composés de formule (XIV) peuvent être préparés par réaction du chlorure de triphénylméthyle sur un ester des formule:

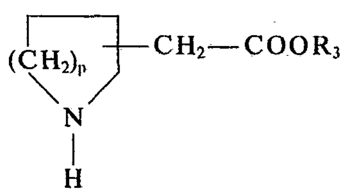

(XVI)

dans laquelle $R_3$ désigne un groupe alkyle de bas poids moléculaire, par exemple méthyle ou éthyle, réduction par l'hydrure de lithium et d'aluminium $LiAlH_4$ de l'ester de formule (XVII) ainsi obtenu et action du chlorure de tosyle sur l'alcool de formule (XVIII) obtenu. L'ensemble des réactions peut être schématisé comme suit:

k)
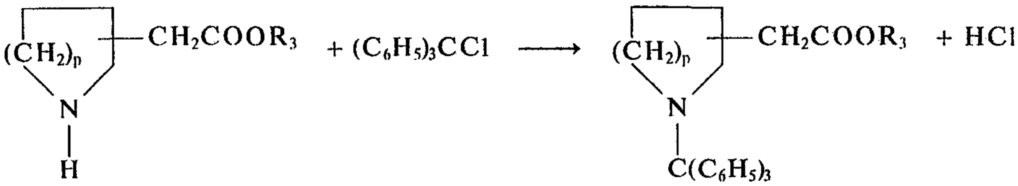


(XVI) (XVII)

e)
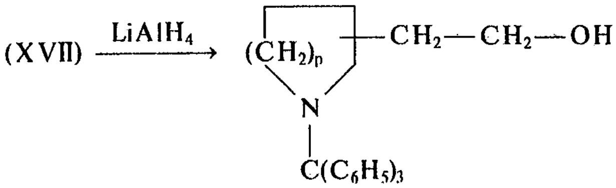


(XVIII)

m)
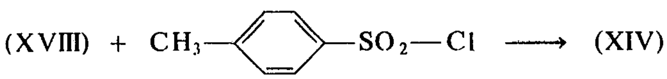


La réaction k) est effectuée avantageusement au sein d'un solvant inerte permettant de solubiliser les réactifs, par exemple le chloroforme, et en présence d'une base telle qu'une amine tertiaire (par exemple la triéthylamine). La réaction 1) est effectuée au sein d'un solvant inerte, de préférence un éther tel que l'oxyde de diéthyle ou le tétrahydrofuranne, à une température comprise entre 0°C et la température ambiante.

Le réaction (m) est effectuée au sein d'un solvant inerte et en présence d'une base, de préférence une amine. Une méthode avantageuse consiste à opérer au sein de la pyridine, qui joue à la fois le rôle de solvant et de base, à une température comprise entre 0°C et 20°C.

Les mélanges réactionnels obtenus par les divers procédés décrits précédemment sont traités suivant des méthodes classiques, physiques (évaporation, extraction à l'aide d'un solvant, distillation, cristallisation, chromatographie, etc.) ou chimiques (formation de sel et régénération de la base, etc.) afin d'isoler les composés de formule (I) à l'état pur.

Les composés de formule (I) sous forme de base libre peuvent éventuellement être transformés en sels d'addition avec un acide minéral ou organique par action d'un tel acide au sein d'un solvant approprié.

Les énantiomères purs répondant à la formule (I) peuvent être obtenus à partir des produits racémiques correspondants par formation de composés diastéréoisomères intermédiaires, séparation des diastéréoisomères formés par recristallisation ou chromatographie, puis régénération à partir de chaque diastéréoisomère du produit de départ sous forme optiquement pure, c'est-à-dire sous forme d'un énantiomère pur.

Comme composés diastéréoisomères intermédiaires on peut utiliser en particulier les sels obtenus par action d'un acide optiquement actif sur les produits racémiques de formule (I).

Dans le cas où $R_1$ est un atome d'hydrogène on peut également utiliser avantageusement comme composés diastéréoisomères intermédiaires les amides N-substitués obtenus par action d'un chlorure d'acide optiquement actif tel que exemple le chlorure de l'acide (-)$\alpha$-méthoxy-$\alpha$-trifluorométhyl phénylacétique sur les produits racémiques de formule (I) pour lesquels $R_1$ = H. Le mélange des amides N-substitués diastéréoisomères obtenu est soumis à une chromatographie et chaque amide diastéréoisomère ainsi isolé est hydrolysé. L'ensemble des réactions peut être schématisé comme suit:

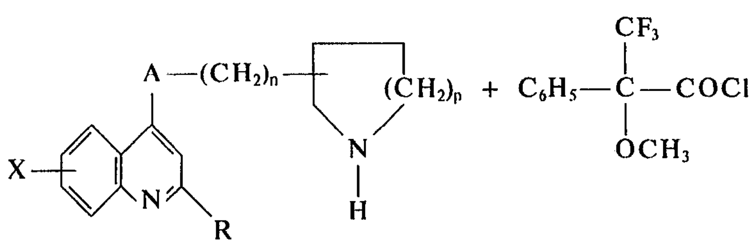

(racémique)

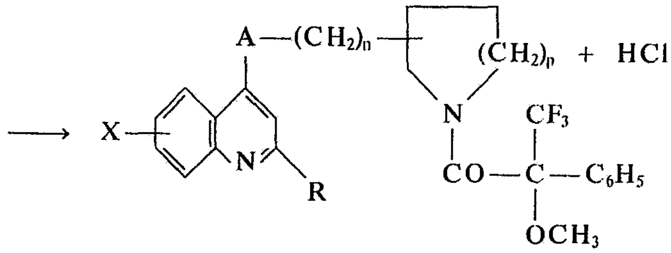

(XIX) (mélange de deux diastéréoisomères A et B)

(XIX) chromatographie →

Diastéréoisomère A —hydrolyse→ (I) avec $R_1 = H$ (énantiomère lévogyre)

Diastéréoisomère B —hydrolyse→ (I) avec $R_1 = H$ (énantiomère dextrogyre)

La réaction du chlorure d'acide optiquement actif sur les produits racémiques de formule (I) pour lesquels $R_1 = H$ est effectuée au sein d'un solvant inerte tel que le chloroforme ou le tétrachlorure de carbone, en présence d'une base telle que la pyridine, à la température ambiante. L'hydrolyse des amides N-substitués est effectués en milieu aqueux, en présence d'une base forte telle que l'hydroxyde de sodium ou de potassium et de préférence en présence d'un solvant auxiliaire à point d'ébullition élevé tel que l'éthylèneglycol ou son éther monoéthylique (cellosolve), à la température d'ébullition du milieu.

Les médicaments de la classe des benzodiazépines sont utilisés comme anticonvulsivants, comme hypnotiques et pour le traitement des états d'anxiété et de divers états psychonévrotiques. La présence de récepteurs spécifiques des benzodiazépines dans les membranes de cerveau de rataété démontrée [Squires et Coll., Nature, 266, (1977), 732] et le degré d'affinité des benzodiazépines pour ces récepteurs, degré d'affinité mesuré par leur aptitude à déplacer de ses sites de liaison le Diazépan tritié, est en bonne corrélation avec les effets pharmacodynamiques observés chez l'animal et chez l'homme.

Les produits de l'invention, bien que de structure différente de celle des benzodiezépines, déplacent le Diazépam de ses sites de liaison. Ils peuvent donc trouver des applications comme hypnotiques, comme anticonvulsivants, et dans le traitement des états de tension et d'anxiété résultant de circonstances »stressantes« ou de troubles somatiques liés à des facteurs émotionnels. Ils sont utilisables pour le traitement des états psychonévretiques se manifestant par des symtômes d'anxiété, d'appréhension, de fatigue, d'agitation ou de dépression.

Les produits de l'invention possèdent d'autre part des propriétés antiarythmiques.

Les exemples suivants illustrent l'invention sans la limiter. Les données relatives aux spectres de résonance magnétique nucléaire (en abrégé: spectres R.M.N.) figurant dans ces exemples concernent la résonance magnétique nucléaire des protons des composés à l'état de base. Pour effectuer les mesures ce résonance magnétique nucléaire les composés sont mis en solution dans le chloroforme deuteré.

### Exemple 1
### (phényl-2 quinolyl-4)-1 (pipéridyl-3)-2 éthanone

A 200 ml de tétrahydrofuranne sec, sous atmosphère d'azote, on ajoute lentement 36 ml d'une suspension à 20% d'hydrure de potassium dans l'huile. On introduit ensuite une solution de 18 g de phényl-2 quinoléinecarboxylate-4 d'éthyle dans 20 ml de tétrahydrofuranne sec, puis on ajoute lentement, en deux heures, une solution de 16,5 g de N-benzoyl pipéridyl-3 acétate d'éthyle dans 100 ml de tétrahydrofuranne sec. On agite 5 heures à température ambiante. On ajoute ensuite 20 ml d'éthanol, élimine le solvant par distillation sous pression réduite, reprend le résidu par 300 ml d'une solution aqueuse 5 N d'acide chlorhydrique et chauffe au reflux pendant 13 heures. On extrait la phase aqueuse par 3 fois 300 ml d'éther, puis l'alcalinise par addition d'ammoniaque concentrée, et extrait par 3 fois 300 ml d'acétate d'éthyle. On lave la phase acétate d'éthyle à l'eau et la sèche sur sulfate de magnésium. Après élimination du solvant par distillation sous pression réduite, on obtient 16,2 g de produit brut qui est absorbé sur une colonne contenant 810 g de silice. Après élution avec un mélange chloroforme-diéthylamine 9/1, on obtient 12,4 g de (phényl-2 quinolyl-4)-1 (pipéridyl-3)-2 éthanone, que l'on transforme en monochlorhydrate. Ce monochlorhydrate fond à 218°C.

Exemple 2

(phényl-2 quinolyl-4)-1 (pipéridyl-3)-3 propanone-1

A une solution de 6 g de phényl-2 quinoléine-carboxylate-4 d'éthyle dans 150 ml de tétrahydrofuranne sec, placée sous atmosphère d'azote, on ajoute, goutte-à-goutte, 12 ml d'une suspension à 20% d'hydrure de potassium dans l'huile. On ajoute ensuite lentement, en deux heures, une solution de 5,8 g de (N-benzoyl pipéridyl-3)-3 propionate d'éthyle dans 50 ml de tétrahydrofuranne sec, et on agite à 25°C pendant 3 heures. On ajoute ensuite 10 ml d'éthanol, élimine le solvant par distillation sous pression réduite, reprend le résidu par 150 ml d'une solution aqueuse 5 N d'acide chlorhydrique et chauffe au reflux pendant 22 heures. On extrait la solution aqueuse par 4 fois 100 ml d'éther, puis l'amène à pH 11 par addition d'hydroxyde de potassium et extrait par 3 fois 200 ml d'acétate d'éthyle. On lave la phase acétate d'éthyle à l'eau et la sèche sur sulfate de magnésium. Après élimination du solvant sous pression réduite, on obtient 3,8 g de produit brut. Ce produit est absorbé sur une colonne contenant 380 g de silice. Après élution avec un mélange chloroforme-diéthylamine 93/7, on obtient 2,3 g de (phényl-2 quinolyl-4)-1 (pipéridyl-3)-3 propanone-1 dont le monochlorhydrate, formé au sein de l'acétone par addition d'une solution 5, 8 N d'acide chlorhydrique dans l'éther, fond à 224°C.

Le (N-benzoyl pipéridyl-3)-3 propionate d'éthyle peut être préparé de la manière suivante:

A une solution agitée de 16,8 g de (pipéridyl-3)-3 propionate d'éthyle (préparé comme indiqué dans le brevet US 3 159 639) dans 150 ml de chloroforme, on ajoute 43 g de carbonate de potassium. On refroidit à 10°C, puis on coule en 15 minutes 16 g de chlorure de benzoyle. On chauffe ensuite au reflux pendant 4 heures. Le milieu réactionnel est évaporé sous pression réduité. Le résidu est repris par une solution aqueuse de bicarbonate de sodium et on extrait à l'acétate d'éthyle. La phase organique est séchée sur sulfate de magnésium et évaporée sous pression réduite. Le résidu est fixé sur une colonne contenant 500 g de silice et on élue avec un mélange cyclohexane-acétate d'éthyle 5/5.

On isole ainsi 15,7 g de (N-benzoyl pipéridyl-3)-3 propionate d'éthyle sous forme d'une huile.

Spectre R.M.N. du produit obtenu:

Les déplacements chimiques $\delta$ des protons sont les suivants:

| | |
|---|---|
| protons du noyau aromatique | $\delta = 7{,}4$ ppm |
| $-C\underline{H}_2-CO-$ | $\delta \simeq 2{,}2$ ppm |
| $-C\underline{H}_2-N-$ (N trisubstitué) | $\delta \simeq 4$ ppm (équatoriaux) <br> $\delta \simeq 2{,}9$ ppm (axiaux) |

Exemple 3

(chloro-6 phényl-2) quinolyl-4)-1 (pipéridyl-3)-2 éthanone

On opère comme à l'exemple 1, en partant de 12,2 g de chloro-6 phényl-2 quinoléinecarboxylate-4 d'éthyle, 8,25 g de N-benzoyl pipéridyl-3 acétat d'éthyle et 18 ml d'une suspension d'hydrure de potassium à 20 %dans l'huile. On obtient 8,1 g de (chloro-6 phényl-2 quinolyl-4)-1 (pipéridyl-3)-2 éthanone. Ce dernier composé est mis en solution dans l'acétone et transformé, par addition d'acide chlorhydrique, en son monochlorhydrate, qui fond au-dessus de 260°C.

Exemple 4

[(trifluorométhyl-3 phényl)-2 quinolyl-4]-1 (pipéridyl-3)-2 éthanone

A 200 ml de tétrahydrofuranne sec, sous atmosphère d'azote, on ajoute lentément 30 ml d'une suspension à 20%d'hydrure de potassium dans l'huile. On porte le mélange au reflux, puis on introduit 19 g de (trifluorométhyl-3 phényl)-2 quinoléinecarboxylate-4 d'éthyle. On ajoute ensuite lentement, en une heure, une solution de 14,7 g de N-benzoyl pipéridyl-3 acétat d'éthyle dans 100 ml de tétrahydrofuranne sec. On agite 2 heures au reflux. On ajoute ensuite 20 ml d'éthanol, puis 20 ml d'eau, on élimine le solvant par distillation sous pression réduite, reprend le résidu par 300 ml d'une solution aqueuse 5 N d'acide chlorhydrique et chauffe au reflux pendant 13 heures. On extrait la phase aqueuse par 2 fois 200 ml d'éther, la filtre, l'alcalinise par addition d'ammoniaque concentrée or l'extrait par 2 fois 200 ml d'acétate d'éthyle. La phase acétate d'éthyle est séchée sur sulfate de magnésium, traitée par 1 g de noir animal, filtrée, et évaporée sous pression réduite. On obtient ainsi 5 g de [trifluorométhyl-3 phényl)-2 quinolyl-4]-1 (pipéridyl-3)-2 éthanone.

Spectre R.M.N. du produit obtenu:

protons du noyau aromatique: $\delta$ = 7,3–8,4 ppm

$-\underset{|}{N}-\underline{CH_2}-$ et $-CO-\underline{CH_2}-$: $\delta$ = 3–4 ppm

## Exemple 5

### [(chloro-4 phényl)-2 quinolyl)-4]-1 (pipéridyl-3)-2 éthanone

On opère comme dans l'exemple 1, mais en partant de 13 g de (chloro-4 phényl)-2 quinoléinecarboxylate-4 d'éthyle, 23 ml d'une suspension d'hydrure de potassium à 20 % dans l'huile et 10,9 g de N-benzoyl pipéridyl-3 acétate d'éthyle. On obtient 6,8 g de produit brut, que l'on fixe sur une colonne contenant 340 g de silice. Après élution avec un mélange chloroforme-diéthylamine 9/1, on isole 3,5 g de [(chloro-4 phényl)-2 quinolyl-4]-1 (pipéridyl-3)-2 éthanone sous forme d'une huile.

Spectre R.M.N. du produit obtenu:

protons du noyau aromatique: $\delta$ = 7,3 à 8,4 ppm

$-\underset{|}{N}-\underline{CH_2}-$ et $-CO-\underline{CH_2}-$: $\delta$ = 3 à 4 ppm

## Exemple 6

### Phényl-2 [(pipéridyl-3)-2 éthyl]-4 quinoléine

On chauffe à 180°C, pendant 15 minutes, un mélange de 11 g de (phényl-2 quinolyl-4)-1 (pipéridyl-3)-2 éthanone et 4,5 ml d'hydrate d'hydrazine à 85 % dans 50 ml de diéthylèneglycol. Après refroidissement à 50°C, on ajoute 6,7 g d'hydroxyde de potassium en pastilles et chauffe à 180°C pendant 3 heures. On dilue le mélange réactionnel avec de l'eau, extrait à l'acétate d'éthyle. La phase organique est séchée sur sulfate de magnésium, décolorée à l'aide de noir animal, filtrée et évaporée sous pression réduite. On obtient ainsi 10,2 g de phényl-2 [(pipéridyl-3)-2 éthyl]-4 quinoléine. Ce dernier composé est mis en solution dans l'acétone et transformé, par addition d'acide chlorhydrique, en son monochlorhydrate, qui fond à 206°C.

## Exemple 7

### phényl-2 [(pipéridyl-3)-3 propyl]-4 quinoléine

On opère comme à l'exemple 6, en partant de 11,1 g de (phényl-2 quinolyl-4)-1 (pipéridyl-3)-3 propanone-1, 11 ml d'hydrate d'hydrazine à 85 % et 16,6 g d'hydroxyde de potassium. On obtient 10,1 g de produit brut, qui sont absorbés sur une colonne de 610 g de silice. Après élution avec un mélange chloroforme-diéthylamine 90/10, on isole 7,6 g de phényl-2 [(pipéridyl-3)-3 propyl]-4 quinoléine, que l'on transforme en son monochlorhydrate. Ce monochlorhydrate fond à 154°C.

## Exemple 8

### chloro-6 phényl-2 [(pipéridyl-3)-2 éthyl]-4 quinoléine

On chauffe à 180°C, pendant 15 minutes, un mélange de 4 g de (chloro-6 phényl-2 quinolyl-4)-1 (pipéridyl-3)-2 éthanone et de 1,9 ml d'hydrate d'hydrazine à 85 % dans 20 ml de diéthylèneglycol. On refroidit à 60°C, puis on ajoute 2,4 g d'hydroxyde de potassium en pastilles, et on chauffe à 120°C pendant 3 heures 30 minutes. On refroidit à 50°C, coule le mélange dans 200 ml d'un mélange d'eau et de glace et extrait par deux fois 100 ml d'acétate d'éthyle. La phase organique est lavée à l'eau, séchée sur sulfate de magnésium et évaporée sous pression réduite. On obtient ainsi 3,4 g de chloro-6- phényl-2 [(pipéridyl-3)-2 éthyl]-4 quinoléine. Ce composé est dissous dans l'acétone et transformé, par addition d'acide chlorhydrique, en son monochlorhydrate. De dernier présente, après recristallisation dans du méthanol à 2 % d'eau, un point de fusion supérieur à 260°C.

## Exemple 9

### [(pipéridyl-3)-2 éthyl]-4 (trifluorométhyl-3 phényl)-2 quinoléine

On chauffe à 160°C, pendant 30 minutes, un mélange de 4,5 g de [trifluorométhyl-3 phényl)-2 quinolyl-4]-1 (pipéridyl-3)-2 éthanone et de 1,8 ml d'hydrate d'hydrazine à 85 % dans 40 ml de diéthylèneglycol. On refroidit à 100°C, puis on ajoute 1,8 g d'hydroxyde de potassium en pastilles et on chauffe à 180°C pendant 4 heures. On dilue le mélange réactionnel avec de l'eau, extrait à l'éther. La phase organique est séchée sur sulfate de magnésium et évaporée sous pression réduite. Le résidu obtenu est fixé sur une colonne de silice et on élue avec un mélange chloroforme-diéthylamine 9/1. On isole ainsi 2,2 g de [(pipéridyl-3)-2 éthyl]-4 (trifluorométhyl-3 phényl)-2 quinoléine. Ce composé est mis en solution dans l'acétone et transformé, par addition d'acide chlorhydrique, en son monochlorhydrate, qui fond à 228°C.

## Exemple 10

### (chloro-4 phényl)-2 [(pipéridyl-3)-2 éthyl]-4 quinoléine

On chauffe à 160°C, pendant 1 heure, un mélange de 3,3 g de [(chloro-4 phényl)-2 quinolyl-4]-1 (pipéridyl-3)-2 éthanone et de 1,5 ml d'hydrate d'hyrazine à 85 % dans 35 ml de diéthylèneglycol. On ajoute ensuite au milieu réactionnel 1 ml d'hydrate d'hydrazine à 85 % et on chauffe à 175°C pendant 1 heure. On refroidit à 100°C, puis on ajoute 1,5 g d'hydroxyde de potassium en pastilles et on chauffe à 175°C pendant 2 heures. On dilue le mélange réactionnel avec de l'eau, extrait au chloroforme. La phase organique est séchée sur sulfate de magnésium et évaporée sous pression réduite. On obtient ainsi 3 g de (chloro-4 phényl)-2 [pipéridyl-3)-2 éthyl]-4 quinoléine. Ce cernier composé est mis en solution dans l'acétone et transformé, par addition d'acide chlorhydrique, en son monochlorhydrate, qui fond à 155°C.

## Exemple 11

### Phényl-2 [(pipéridyl-2)-2 éthyl]-4 quinoléine

1) Préparation de la (pyridyl-2)-2 (phényl-2 quinolyl-4)-1 éthanone.

A 200 ml de tétrahydrofuranne sec, placés sous atmosphère d'azote, on ajoute 40,5 g de diisopropylamine. La solution est agitée, puis refroidie à −60°C. On introduit alors en 10 minutes 200 ml d'une solution à 15 % de butyllithium dans l'hexane. Quand la température s'est stabilisée à −60°C, on introduit en 10 minutes une solution de 28 g de méthyl-2 pyridine dans 100 ml de tétrahydrofuranne sec. On refroidit à −60°C, puis on introduit en 10 minutes une solution de 27,7 g de phényl-2 quinoléinecarboxylate-4 d'éthyle dans 100 ml de tétrahydrofuranne sec. On agite le milieu réactionnel 20 minutes à −60°C, puis on introduit goutte-à-goutte 100 ml d'éthanol. On ramène la température à −20°C, puis on introduit goutte-à-goutte 100 ml d'eau, et on laisse deux heures sous agitation. On filtre le précipité obtenu, le lave avec 3 fois 300 ml d'eau, puis avec trois fois 100 ml d'acétone et enfin avec 100 ml d'éther. On obtient ainsi 22 g de (phényl-2 quinolyl-4)-1 (pyridyl-2)-2 éthanone, qui fond vers 250°C.

2) Préparation de la phényl-2 [(pyridyl-2)-2 éthyl]-4 quinoléine

On chauffe à 180°C pendant 1 heure et 15 minutes un mélange de 13 g de (phényl-2 quinolyl-4)-1 (pyridyl-2)-2 éthanone et de 2,3 ml d'hydrate d'hydrazine à 85 % dans 40 ml de diéthylèneglycol. On refroidit à température ambiante, puis on ajoute 2 g d'hydroxyde de sodium en pastilles et on chauffe à 180°C pendant 5 heures. Le mélanges réactionnel est dilué à l'eau puis extrait au chloroforme. La phase organique est séchée sur sulfate de magnésium et évaporée sous pression réduite. On obtient ainsi 5 g d'un résidu huileux, que l'on fixe sur une colonne contenant 500 g de silice. On élue ensuite avec un mélange chloroforme/cyclohexane/diéthylamine 5/4/1 sous une pression de 5 bars. On isole ainsi 3 g de phényl-2 [(pyridyl-2)-2 éthyl]-4 quinoléine sous forme d'une huile.

Spectre R.M.N. du produit obtenu:

| | |
|---|---|
| protons du noyau aromatique: | $\delta = 7$ à 8,2 ppm |
| $\underline{CH_2}$—Ar: | $\delta = 3$ à 3,6 ppm |

3) Préparation de la phényl-2 [(pipéridyl-2)-2 éthyl]-4 quinoléine

A une solution contenant 2,5 g de phényl-2 [(pyridyl-2)-2 éthyl]-4 quinoléine dans 250 ml d'éthanol, on ajoute 4,55 ml d'acide acétique concentré, puis 0,5 g d'oxyde de platine à 82 % à titre de catalyseur. On hydrogène à 30°C, sous une pression d'hydrogène égale à la pression normale, pendant 6 heures, temps au bout duquel 700 ml d'hydrogène ont été absorbés. Le catalyseur est éliminé par filtration, l'éthanol évaporé sous pression réduite et le résidu repris par 200 ml d'eau. On alcalinise la phase aqueuse à l'aide d'ammoniaque concentré, et on extrait au chlorure de méthylène. La phase organique est séchée sur sulfate de magnésium et évaporée sous pression réduite. Le résidu obtenu est fixé sur une colonne de 200 g de silice et on élue avec un mélange toluène-diéthylamine 9/1. On isole ainsi 0,65 g de phényl-2 [pipéridyl-2)-2 éthyl]-4 quinoléine sous forme d'une huile. Ce composé est mis en solution dans l'acétone et transformé, par addition d'acide chlorhydrique, en son dichlorhydrate, qui fond à 200°C.

Exemple 12

[(méthyl-1 pipéridyl-3)-2 éthyl]-4 phényl-2 quinoléine

Un mélange contenant 2,8 g de phényl-2 [(pipéridyl-3)-2 éthyl]-4 quinoléine, 20 ml d'acide formique et 20 ml de solution aqueuse de formaldéhyde à 37 % est chauffé à 95—100°C pendant 2 heures 30 minutes. Après refroidissement à la température ambiante, le milieu réactionnel est coulé dans un litre d'eau, puis on extrait avec un litre d'acétate d'éthyle. La phase organique est séchée sur sulfate de magnésium et évaporée sous pression réduite. Le résidu est fixé sur une colonne de 500 g de silice et élue avec un mélange chloroforme-diéthylamine 95/5, sous une pression de 5 bars. On obtient ainsi 1,6 g de [(méthyl-1 pipéridyl-3)-2 éthyl]-4 phényl-2 quinoléine. Ce composé est mis en solution dans l'acétone et transformé, par addition d'acide chlorhydrique, en son monochlorhydrate, qui fond à 177°C.

Exemple 13

[(méthyl-1 pipéridyl-3)-3 propyl]-4 phényl-2 quinoléine

On opère comme à l'exemple 12, en partant de 3,8 g de phényl-2 [(pipéridyl-3)-3 propyl]-4 quinoléine, 35 ml d'acide formique et 35 ml de solution aqueuse de formaldéhyde à 37 %. Le produit brut (3 g) obtenu après extraction à l'acétate d'éthyle et évaporation du solvant est fixé sur une colonne contenant 400 g de silice et on élue avec un mélange chloroformediéthylamine 90/10. On obtient ainsi 1,6 g de [(méthyl-1 pipéridyl-3)-3 propyl]-4 phényl-2 quinoléine, dont le monochlorhydrate fond à 163°C.

Exemple 14

(phényl-2 quinolyl-4)-1 (pyrrolidinyl-3)-2 éthanone-1

1) Préparation du N-benzoyl pyrrolidinyl-3 acétate de méthyle.

A une solution agitée de 38,1 g d'acétate de pyrrolidinyl-3 acétate de méthyle dans 800 ml de chloroforme, on ajoute 104 g de carbonate de potassium anhydre. On agite pendant une heure à la température ambiante, puis refroidit à 10°C et introduit lentement, en l'espace de 30 minutes, 36 ml de chlorure de benzoyle. On laisse 12 heures sous agitation. Les sels minéraux sont éliminés par filtration et lavés au chloroforme. Les phases chloroformiques sont rassemblées, lavées successivement avec 250 ml d'eau, 250 ml d'une solution aqueuse normale d'acide chlorhydrique, 250 ml d'une solution aqueuse à 5 % de bicarbonate de sodium et enfin 250 ml d'eau, séchées sur sulfate de magnésium et évaporées sous pression réduite. Le résidu obtenu est fixé sur une colonne contenant 800 g de silice et on élue avec de l'acétate d'éthyle. On isole ainsi 14,3 g de N-benzoyl pyrrolidinyl-3 acétate de méthyle.

Spectre R.M.N. du produit obtenu:

| | |
|---|---|
| protons du noyau aromatique: | $\delta = 7{,}4$ ppm |
| $-\underline{CH_2}-CO$: | $\delta = 2{,}4$ ppm |
| $-\underline{CH_2}-\underset{\vert}{N}-CO$: | $\delta = 3$ à $4$ ppm |

L'acétate de pyrrolidinyl-3 acétate de méthyle peut être préparé par le procédé décrit dans le brevet japonais 4820/59.

2) Préparation de la (phényl-2 quinolyl-4)-1 (pyrrolidinyl-3)-2 éthanone-1

A 140 ml de tétrahydrofuranne sec, placés sous atmosphère d'azote, on ajoute lentement 48 ml d'une suspension à 20 % d'hydrure de potassium dans l'huile. On introduit ensuite 11,6 g de phényl-2 quinoléinecarboxylate-4 d'éthyle, puis on ajoute lentement, en l'espace de deux heures, une solution de 8,6 g de N-benzoyl pyrrolidinyl-3 acétate de méthyle dans 70 ml de tétrahydrofuranne sec. On agite pendant 1 heure et 30 minutes à la température ambiante. On ajoute ensuite 35 ml d'éthanol absolu, élimine le solvant par distillation sous pression réduite. On reprend le résidu par 175 ml d'une solution aqueuse 5 N d'acide chlorhydrique et chauffe au reflux pendant 13 heures. On extrait la phase aqueuse par deux fois 200 ml d'éther, puis on l'amène à sec. Le résidu obtenu est lavé plusieurs fois à l'acétone. On obtient ainsi 13,3 g de (phényl-2 quinolyl-4)-1 (pyrrolidinyl-3)-2 éthanone-1 sous forme de dichlorhydrate.

Spectre R.M.N. du produit obtenu:

protons du noyau aromatique: $\delta =$ 7,3 à 8,4 ppm

$-CH_2-CO$ et $\underline{CH_2}-\overset{|}{N}-$: $\delta =$ 2,5 à 3,2 ppm

Exemple 15

phényl-2 [(pyrrolidinyl-3)-2 éthyl]-4 quinoléine

On chauffe à 160°C pendant 15 minutes un mélange de 13,3 g de dichlorhydrate de (phényl-2 quinolyl-4)-1 (pyrrolidinyl-3)-2 éthanone-1 et de 8,3 g d'hydrate d'hydrazine à 85 % dans 40 ml de diéthylèneglycol. On refroidit à 100°C, puis on ajoute par portions 9,8 g d'hydroxyde de potassium en pastilles, et on chauffe à 160°C pendant 5 heures. On dilue le mélange réactionnel à l'eau, extrait par 3 fois 100 ml de chlorure de méthylène. La phase organique est séchée sur sulfate de magnésium et évaporée sous pression réduite. Le résidu obtenu est absorbé sur une colonne de 250 g de silice et on élue avec un mélange chloroforme-diéthylamine 90/10. On isole ainsi 5,5 g de phényl-2 [(pyrrolidinyl-3)-2 éthyl]-4 quinoléine sous forme d'une huile. Ce composé est mis en solution dans l'isopropanol et transformé, par addition d'acide chlorhydrique, en son monochlorhydrate qui fond à 140°C.

Exemple 16

[(pipéridyl-2)-3 propyl-1]-4 phényl-2 quinoléine

1) Préparation du (triphénylméthyl-1 pipéridine-2) acétate d'éthyle

A une solution agitée de 33,8 g de pipéridine-2 acétate d'éthyle dans 700 ml de chloroforme, on ajoute 31 ml de triéthylamine. On refroidit à 0°C, puis on ajoute lentement, par portions, 72,6 g de chlorure de triphénylméthyle. On laisse la température remonter à 20°C et on laisse 18 heures sous agitation. On ajoute alors 18,2 g de chorure de triphénylméthyle ainsi que 8 ml de triéthylamine. On agite pendant 3 heures, puis on ajoute encore 9 g de chlorure de triphénylméthyle et 4 ml de triéthylamine. Le mélange réactionnel est évaporé sous pression réduite et le résidu est repris par 600 ml d'éther. On filtre le précipité de chlorhydrate de triéthylamine formé et évapore le filtrat. On obtient ainsi un résidu huileux jaune que l'on reprend par de l'éther de pétrole. Les cristaux obtenus sont filtrés. On obtient ainsi le (triphénylméthyl-1 pipéridine-2) acétate d'éthyle, qui fond à 95°C.

2) Préparation du (triphénylméthyl-1 pipéridine-2) éthanol.

A 600 ml de tétrahydrofuranne anhydre, placés sous atmosphère d'azote, on ajoute lentement, par portions, 15,2 g d'hydrure d'aluminium et de lithium. La suspension agitée est refroidie à 0°C et on y ajoute lentement, en l'espace d'une heure, 82 g de (triphénylméthyl-1 pipéridine-2) acétate d'éthyle. On laisse la température revenir à la température ambiante et on agite le milieu réactionnel pendant deux heures. On refroidit ensuite à 0°C et ajoute successivement 17 ml d'eau, 12,6 ml d'une solution aqueuse 5 N d'hydroxyde de sodium et 59 ml d'eau. Les produits minéraux sont séparés par filtration et lavés avec du chlorure de méthylène bouillant. Le filtrat et les lavages sont rassemblés, séchés sur sulfate de magnésium et évaporés. On obtient ainsi 127,5 g d'un résidu huileux. Ce résidu est fixé sur une colonne de 1200 g de silice et on élue avec un mélange cyclohexaneacétate d'éthyle 80/20. On isole ainsi 36,5 g de (triphénylméthyl-1 pipéridine-2) éthanol, qui fond à 165°C.

3) Préparation de la (p-tolylsulfonyloxy-2 éthyl]-2 triphénylméthyl-1 pipéridine

A une solution de 15 g de (triphénylméthyl-1 pipéridine-2) éthanol dans 150 ml de pyridine, refroidie à 0°C, on ajoute d'un coup 15 g de chlorure de p-méthylphénylsulfonyle. On laisse deux heures sous agitation à 0°C, puis on coule lentement, sous agitation, le mélange réactionnel dans 1500 ml d'eau. Le précipité obtenu est filtré, lavé à l'eau, séché. On obtient ainsi 17,9 g de (p-tolylsulfonyloxy-2 éthyl)-2 triphénylméthyl-1 pipéridine.

Spectre R.M.N. du produit obtenu:

| | |
|---|---|
| protons des noyaux aromatiques: | $\delta = 7$ à 7,6 ppm |
| $-\underline{CH_2}-O$: | $\delta = 3,6$ ppm |
| $-\underline{CH_2}-\overset{\vert}{N}-$: | $\delta = 2,8$ à 3,2 ppm |

4) Préparation de la [(pipéridyl-2)-3 propyl-1]-4 phényl-2 quinoléine

A 40 ml de tétrahydrofuranne sec, placés sous atmosphère d'azote, on ajoute 5,4 ml de diisopropylamine. La solution est agitée, refroidie à −60°C et on introduit, en l'espace de 15 minutes, 15 ml d'une solution 2 M de butyllithium dans l'hexane. Après stabilisation de la température à −60°C, on introduit une solution de 6,6 g de phényl-2 méthyl-4 quinoléine dans 20 ml de tétrahydrofuranne sec. On laisse 20 minutes sous agitation à −60°C, puis on introduit lentement une solution de 10,5 g de (p-tolylsulfonyloxy-2 éthyl)-2 triphénylméthyl-1 pipéridine dans 50 ml de tétrahydrofuranne sec. On réchauffe jusqu' à la température ambiante et on laisse 3 heures et 30 minutes sous agitation. On ajoute ensuite 50 ml d'eau, évapore sous pression réduite et reprend le résidu par 200 ml d'ether et 50 ml d'eau. La phase éthérée est décantée, lavée à l'eau, séchée sur sulfate de magnésium et concentrée.

Le résidu obtenu est repris par 200 ml d'une solution aqueuse N d'acide chlorhydrique. On agite 10 minutes à la température ambiante, puis la phase aqueuse est lavée par deux fois 200 ml d'éther, amenée à pH 10 par addition d'une solution concentrée d'hydroxyde de sodium et extraite par de l'acétate d'éthyle. L'extrait à l'acétate d'éthyle est évaporé sous pression réduite.

Le résidu obtenu est fixé sur une colonne contenant 400 g de silice et on élue avec un mélange cyclohexane/chloroforme/diéthylamine 70/20/10. On isole ainsi 2,1 g de [(pipéridyl-2)-3 propyl-1]-4 phényl-2 quinoléine sous forme d'une huile. Le monochlorhydrate de ce composé fond à 184°C.

Exemple 17

(pipéridyl-3)-2 [(pyridyl-3)-2 quinolyl-4]-1 éthanone

On opère comme à l'exemple 4, en partant de 10 g de (pyridyl-3)-2 quinoléine-carboxylate-4 d'éthyle, 8,2 g de N-benzoyl pipéridyl-3 acétate d'éthyle et 18 ml d'une suspension d'hydrure de potassium à 20% dans l'huile. On obtient 7,6 g de (pipéridyl-3)-2 [(pyridyl-3)-2 quinolyl-4]-1 éthanone.

Exemple 18

(pipéridyl-3)-2 [(pyridyl-2)-2 quinolyl-4]-1 éthanone

On opère comme à l'exemple 4, en partant de 9,4 g de (pyridyl-2)-2 quinoléine-carboxylate-4 d'éthyle, 7,9 g de N-benzoylpipéridyl-3 acétate d'éthyle et 17,2 ml d'une suspension d'hydrure de potassium à 20% dans l'huile. On obtient 6,9 g de (pipéridyl-3)-2 [(pyridyl-2)-2 quinolyl-4]-1 éthanone.

Exemple 19

[(pipéridyl-3)-2 éthyl]-4 (pyridyl-3)-2 quinoléine

On opère comme à l'exemple 6, en partant de 7,6 g de (pipéridyl-3)-2 [(pyridyl-3)-2 quinolyl-4]-1 éthanone, 4,1 ml d'hydrate d'hydrazine à 85% et 3,9 g d'hydroxyde de potassium. On obtient 6,8 g de [(pipéridyl-3)-2 éthyl]-4 (pyridyl-3)-2 quinoléine, que l'on transforme au sein de l'isopropanol en son monochlorhydrate.

Ce dernier présente, après recristallisation dans l'éthanol, un point de fusion de 165°C.

Exemple 20

[(pipéridyl-3)-2 éthyl]-4 (pyridyl-2)-2 quinoléine

On procède comme à l'exemple 6, en partant de 6 g de (pipéridyl-3)-2 [(pyridyl-2)-2 quinolyl-4]-1 éthanone, 3,2 g d'hydrate d'hydrazine à 85 % et 4,48 g d'hydroxyde de potassium. On obtient 5,5 g de [(pipéridyl-3)-2 éthyl]-4 (pyridyl-2)-2 quinoléine que l'on transforme au sein de l'isopropanol en son monochlorhydrate, qui fond, après recristallisation dans l'éthanol, à 217°C.

Exemple 21

Phényl-2 [(pipéridyl-3)-2 éthyl]-4 quinoléine, énantiomères dextrogyre et lévogyre

1) préparation des {[(α-méthoxy α-trifluorométhyl phényl-acétyl)-1 pipéridyl-3]-2 éthyl}-4 phényl-2 quinoléine diastéréoisomères

A une solution agitée de 12,8 g de phényl-2 [(pipéridyl-3)-2 éthyl]-4 quinoléine (obtenue comme indiqué à l'exemple 6) dens 20 ml de tétrachlorure de carbone, on ajoute 25,5 ml de pyridine anhydre, puis on introduit, en 5 minutes, une solution de 23 g de chlorure de l'acide (-)α-trifluorométhyl phénylacétique dans 20 ml de tétrachlorure de carbone. On agite à température ambiants pendant 1 heure. Après évaporation sous pression réduite, on reprend le résidu par 200 ml de chlorure de méthylène et 100 ml d'eau.

La phase organique est séparés par décantation, lavée par 2 fois 100 ml d'une solution diluée de carbonate de potassium et séchée sur sulfate de magnésium. On obtient, après concentration sous pression réduite, 21 g d'un mélange de {[(α-méthoxy α-trifluorométhyl phénylacétyl)-1 pipéridyl-3]-2 éthyl}-4 phényl-2 quinoléine diastéréoisomères.

Ce mélange est fixé sur une colonne de silice de 500 g et élué sous pression avec un mélange cyclohexane/éther isopropylique/éther éthylique 50/30/20.

On obtient ainsi dans l'ordre d'élution 8,2 g de diastéréoisomère A et 6,2 g de diastéréoisomère 8 purs.

Le chlorure de l'acide (-)α-méthoxy α-trifluorométhyl phénylacétique peut être préparé comme indiqué par Dale JA, Dull DL et Mosher HS, J. Org. Chem., 34, (9), 2543—48, (1969).

2) préparation de la phényl-2 [(pipéridyl-3)-2 éthyl]-4 quinoléine énantiomère lévogyre)

A une solution agitée de 7,5 g du diastéréoisomere A décrit ci-dessus dans 75 ml de cellosolve, on ajoute 38 ml d'une solution aqueuse 1 0N d'hydroxyde de sodium, puis 22,5 g d'hydroxyde de sodium en pastilles. On chauffe 20 heures au reflux. Après refroidissement, le mélange réactionnel est dilué par 750 ml d'eau, et extrait par 3 fois 200 ml d'éther. La phase organique est lavée à l'eau, puis extraite par 4 fois 80 ml d'une solution aqueuse N d'acide acétique. La phase aqueuse est amenée à pH = 10 par addition d'une solution concentrée d'hydroxyde de sodium et extraite par 2 fois 100 ml d'acétate d'éthyle. La phase organique est lavée à l'eau, séchée sur sulfate de magnésium et évaporée sous pression réduite. On obtient ainsi 3,5 g de phényl-2 [(pipéridyl-3)-2 éthyl]-4 quinoléine lévogyre, que l'on transforme au sein de l'acétone en son monochlorhydrate qui fond à 197°C.

Pouvoir rotatoire du monochlorhydrate (mesuré sur une solution à 2 % dans HCl N): $[\alpha]_D^{24} = -4 \pm 1,6°$.

3) préparation de la phényl-2 [(pipéridyl-3)-2 éthyl]-4 quinoléine (énantiomère dextrogyre)

On procède comme pour la préparation de l'énantiomère lévogyre, en partant de 5,7 g de diastéréoisomère B, 57 ml de cellosolve, 29 ml d'une solution aqueuse 1 0N d'hydroxyde de sodium, et 17,1 g d'hydroxyde de sodium en pastilles.

On obtient ainsi 2,5 g de phényl-2 [(pipéridyl-3)-2 éthyl]-4 quinoléine dextrogyre, que l'on transforme au sein de l'acétone en son monochlorhydrate qui fond à 197°C et dont le pouvoir rotatoire (mesuré sur une solution à 1,8 % dans HCl N) est: $[\alpha]_D^{24} = -3,8 \pm 1,8°$.

Propriétés pharmacologiques

1. Affinité pour les sites récepteurs cérébraux des benzodiazepines

Cette affinité est mesurée par l'aptitude des produits à déplacer le Diazépam tritié ($^3H$ Diazépam) de son site de liaison et est exprimée par une valeur $K_i$, en micromoles, qui est calculée par la formule:

$$K_i = \frac{IC_{50}}{1+\frac{C}{K_D}}$$

dans laquelle C représente la concentration de $^3H$ Diazépam, $K_D$ une constante d'affinité égale à 2,74 $\mu M$ et $IC_{50}$ la concentration nécessaire pour obtenir une inhibition de 50 %de la liaison du $^3H$ Diazépam.

Les produits ont été testés en employant la méthode de Mohler et Coll., Life Sciences, 20, 2101 (1977) et les résultats obtenus sont rassemblés dans le tableau suivant:

| Produits | $K_i$ ($\mu M$) |
|---|---|
| Exemple 2 | 0,8 |
| Exemple 6 | 0,1 |
| Exemple 7 | 1 |
| Exemple 10 | 1,6 |
| Exemple 11 | 0,6 |
| Exemple 12 | 0,9 |
| Exemple 13 | 3 |
| Exemple 15 | 0,7 |
| Exemple 16 | 2,7 |

## 2. Activité Antiarythmique

L'activité antiarythmique des composés de la présente invention a été démontrée à l'aide du test à l'aconitine chez le rat.

Le principe de la technique repose sur le temps d'induction des arythmies ventriculaires provoquées par l'aconitine en perfusion lente chez le rat. Une substance antiarythmique retarde l'apparition des arythmies et le délai est proportionnel à l'activité de la molécule.

On utilise des groupes de 5 rats mâles. Une anesthésie individuelle est réalisée (uréthane 10%: 1 g/kg/ip) pour permettre une cathétérisation de la veine du pénis. L'électrocardiogramme est enregistré. Au temps T = 0 la substance étudiée est injectée sous forme d'une solution aqueuse, à raison de 2,5 ml de solution par kg en 30 secondes. Au temps T = 90 secondes, soit 1 minute après la fin de l'injection, l'aconitine est perfusée à raison de 20 $\mu g$ par minute, jusqu' à l'apparition d'extra systoles supra-ventriculaires. Le temps de perfusion de l'aconitine est noté.

On exprime les résultats par une $DE_{50}$, dose de produit en mg/kg qui, par rapport aux animaux témoins, augmente de 50% le temps de perfusion de l'aconitine.

Les résultats obtenus sont rassemblés dans le tableau suivant:

| Produits | $DE_{50}$ mg/kg (i.v.) |
|---|---|
| Exemple 1 | 2,8 |
| Exemple 7 | 2,2 |

## Propriétés toxicologiques

Les toxicités aiguës des composés selon l'invention ont été déterminées chez la souris mâle $CD_1$ (Charles River) par voie orale. Les $DL_{50}$ ont été calculés, après 3 jours d'observation, par la méthode cumulative de J.J. Reed et H. Muench (Amer. J. Hyg. 1938, 27, 493).

Les composés selon l'invention se comportent comme des substances relativement peu toxiques chez la souris, puisque les $DL_{50}$ des composés se situent entre 200 et 1000 mg/kg.

Utilisation thérapeutique

Les composés de l'invention et leurs sels pharmaceutiquement acceptables peuvent être utilisés en thérapeutique humaine, sous forme de comprimés, capsules, gélules, suppositoires, solutions ingérables ou injectables, etc. comme antiarythmiques, hypnotiques, anticonvulsivants et pour le traitement des états d'anxiété et de divers états psychonévrotiques.

La posologie dépend des effets recherchés et de la voie d'administration utilisée. Par exemple, par voie orale, elle peut être comprise entre 5 et 250 mg de substance active par jour, avec des doses unitaires allant de 1 à 50 mg.

**Revendications**

1. Composés de formule générale:

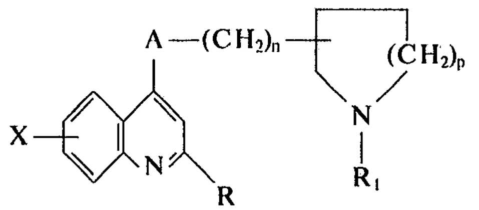

(I)

dans laquelle R représente un groupe phényle, pyridyle ou un groupe phényle substitué par un ou deux substituants pris parmi les atomes d'halogène (chlore, fluor, brome) et groupe trifluorométhyle, X est fixé en position 6 sur le cycle de la quinoléine et représente un atome d'hydrogène ou bien un atome d'halogène (chlore, fluor, brome) ou un groupe méthoxy, $R_1$ représente un atome d'hydrogène, un groupe alkyle comportant 1 à 4 atomes de carbone, A représente un groupe CO ou $CH_2$, n est un nombre entier égal à 1 ou 2, p est un nombre entier égal à 1 ou 2, et le groupe

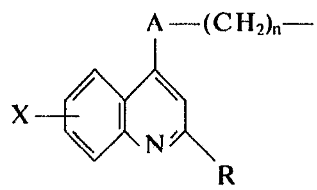

est fixé en position 2 ou 3 sur l'hétérocycle azoté

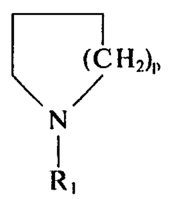

leurs diastéréoisomères, racémiques et énantiomères, et leurs sels d'addition avec les acides minéraux ou organiques.

2. Composés selon la revendication 1 dans lesquels X est un atome d'hydrogène, R est un groupe phényle et n est égal à 1.

3. Composé selon la revendication 2 de formule:

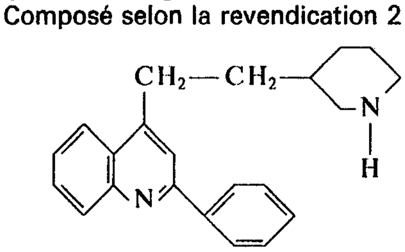

et ses sels d'addition avec les acides minéraux ou organiques.

4. Procédé de préparation des composés selon la revendication 1 pour lesquels A est un groupe $CH_2$, caractérisé en ce que l'on réduit les composés selon la revendication 1 correspondants pour lesquels A est un groupe CO.

5. Procédé de préparation des composés selon la revendication 1 pour lesquels A représente le groupe CO et $R_1$ l'atome d'hydrogène, caractérisé en ce que l'on condense un ester d'acide quinoléine-carboxylique de formule:

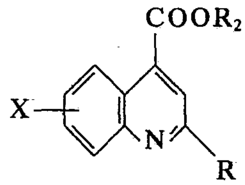

(II)

dans laquelle X et R ont les mêmes significations que dans la formule (I) de la revendication 1 et $R_2$ désigne un groupe alkyle de bas poids moléculaire, avec un ester de formule:

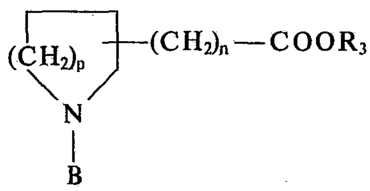

(III)

dans laquelle n et p ont la même signification que dans la formule (I) de la revendication 1, $R_3$ désigne un groupe alkyle de bas poids moléculaire et B un groupe protecteur de la fonction amine et hydrolyse le produit de condensation ainsi obtenu.

6. Procédé de préparation des composés selon la revendication 1 pour lesquels $R_1$ est un groupe alkyle, caractérisé en ce que l'on fait réagir les composés selon la revendication 1 correspondants pour lesquels $R_1$ est un atome d'hydrogène avec un agent alkylant.

7. Procédé de préparation des composés selon la revendication 1 pour lesquels $R_1$ est le groupe méthyle et A le groupe $CH_2$, caractérisé en ce que l'on fait réagir les composés selon la revendication 1 correspondants pour lesquels $R_1$ est un atome d'hydrogène avec le formol au sein de l'acide formique.

8. Procédé pour la préparation des composés selon la revendication 1 pour lesquels A est le groupe $CH_2$ et $R_1$ un groupe alkyle ayant 2 à 4 atomes de carbone caractérisé en ce que l'on fait réagir les composés selon la revendication 1 correspondants pour lesquels A est le groupe $CH_2$ et $R_1$ l'atome d'hydrogène avec un chlorure d'acide de formule

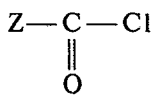

dans laquelle Z est un groupe alkyle ayant 1 à 3 atomes de carbone, et réduit ensuite au moyen d'un hydrure le composé ainsi obtenu.

9. Procédé de préparation des composés selon la revendication 1 répondant à la formule:

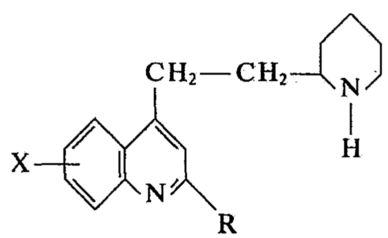

(VII)

caractérisé en ce que l'on hydrogène catalytiquement les dérivés pyridiniques de formule:

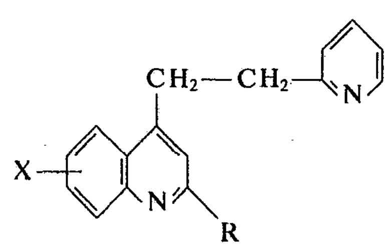

(VIII)

dans laquelle X et R ont la même signification que dans la formule (I) de la revendication 1.

10. Procédé de préparation des composés selon la revendication 1 répondant à la formule:

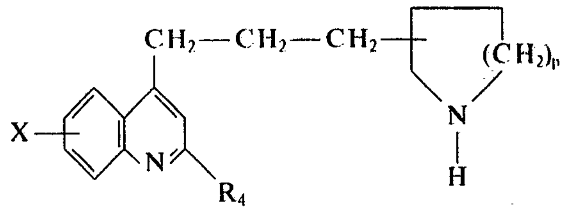

(XI)

dans laquelle X et p ont la même signification que dans la formule (I) de la revendication 1 et $R_4$ désigne un groupe phényle ou un groupe phényle substitué par un ou deux substituants pris parmi les atomes d'halogène $CF_3$, caractérisé en ce que l'on fait réagir un dérivé métallique de formule R"M, dans laquelle M désigne un métal alcalin et R" un atome d'hydrogène, un groupe $NH_2$, un groupe amino disubstitué ou un groupe alkyle ou aryle, avec un dérivé de la quinoléine de formule:

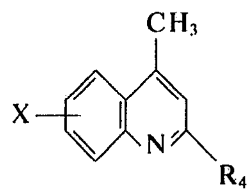

(XII)

dans laquelle X et $R_4$ ont la même signification que dans la formule (XI), fait réagir le composé de formule:

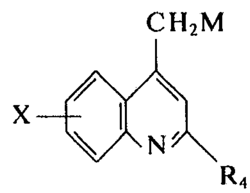

(XIII)

ainsi obtenu avec un composé de formule:

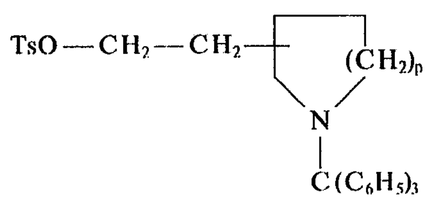

(XIV)

dans laquelle p a la même signification que dans la formule (XI) et Ts désigne le groupe tosyle, et soumet le composé de formule:

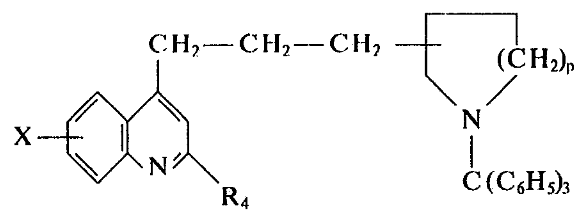

(XV)

ainsi obtenu à l'action d'un acide.

11. Médicament pour utilisation comme antiarythmique, hypnotique, anticonvulsivant et dans le traitement des états d'anxiété et de divers états psychonévrotiques, caractérisé en ce qu'il contient, en tant que principe actif, un composé répondant à la formule (I) de la revendication 1 ou un sel d'un tel composé avec un acide pharmaceutiquement acceptable.

12. Médicament selon la revendication 11, caractérisé en ce qu'il contient, comme principe actif, le composé de formule:

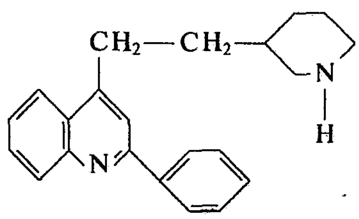

ou un sel de ce composé avec un acide pharmaceutiquement acceptable.

**Patentansprüche**

1. Verbindungen der allgemeinen Formel (I)

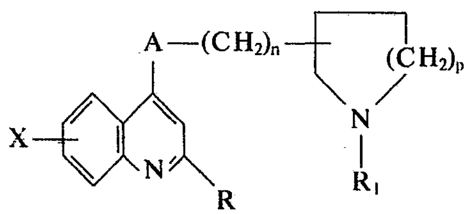

(I)

1. Verbindungen der allgemeinen Formel (I)
worin R eine Phenyl-, Pyridyl- oder eine Phenylgruppe substituiert durch einen oder zwei Substituenten ausgewählt unter den Halogenatomen (Chlor, Fluor, Brom) und der Trifluormethylgruppe substituiert ist, X in 6-Stellung auf dem Chinolinring fixiert ist und ein Wasserstoffatom oder auch ein Halogenatom (Chor, Fluor, Brom) oder eine Methoxygruppe bedeutet, $R_1$ ein Wasserstoffatom, eine Alkylgruppe mit 1 bis 4 Kohlenstoffatomen ist, A eine Gruppe CO oder $CH_2$ ist und n eine ganze Zahl gleich 1 oder 2, p eine ganze Zahl gleich 1 oder 2 ist und die Gruppe

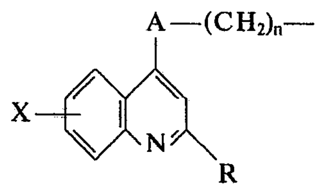

in 2- oder 3-Stellung auf dem Stickstoffheterocyclus

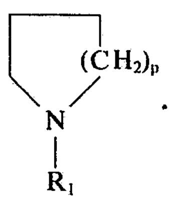

fixiert ist, ihre Diastereoisomeren, Razemate und Enantiomeren und ihre Säureadditionssalze mit Mineralsäuren oder organischen Säuren.

2. Verbindungen gemäß Anspruch 1, worin X ein Wasserstoffatom, R eine Phenylgruppe und n = 1 ist.

3. Verbindung gemäß Anspruch 2 der Formel

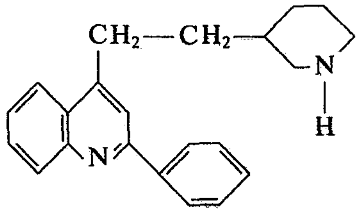

und ihre Additionssalze mit Mineralsäuren oder organischen Säuren.

4. Verfahren zur Herstellung der Verbindungen gemäß Anspruch 1, für die A eine $CH_2$-Gruppe ist, dadurch gekennzeichnet, daß man die entsprechenden Verbindungen gemäß Anspruch 1, für die A eine CO-Gruppe ist, reduziert.

5. Verfahren zur Herstellung der Verbindungen gemäß Anspruch 1, für die A die Gruppe CO bedeutet und $R_1$ ein Wasserstoffatom ist, dadurch gekennzeichnet, daß man einen Chinolincarbonsäureester der Formel (II)

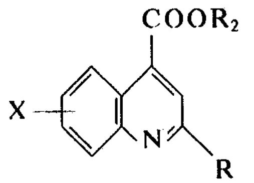

(II)

worin X und R dieselben Bedeutungen wie in Formel (I) von Anspruch 1 haben und $R_2$ eine Alkylgruppe mit niedrigem Molekulargewicht bedeutet, mit einem Ester der Formel (III)

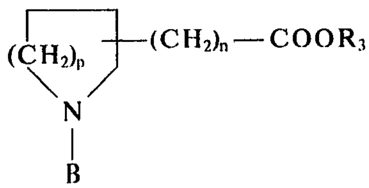

(III)

worin n und p dieselbe Bedeutung wie in Formel (I) von Anspruch 1 haben, $R_3$ eine Alkylgruppe mit niedrigem Molekulargewicht und B eine Schutzgruppe der Aminfunktion bedeutet, kondensiert und das so erhaltene Kondensationsprodukt hydrolysiert.

6. Verfahren zur Herstellung der Verbindungen gemäß Anspruch 1, für die $R_1$ eine Alkylgruppe ist, dadurch gekennzeichnet, daß man die entsprechenden Verbindungen gemäß Anspruch 1, für die $R_1$ ein Wasserstoffatom ist, mit einem Alkylierungsmittel zur Reaktion bringt.

7. Verfahren zur Herstellung der Verbindungen gemäß Anspruch 1, für die $R_1$ die Methylgruppe und A die $CH_2$-Gruppe ist, dadurch gekennzeichnet, daß man die entsprechenden Verbindungen gemäß Anspruch 1, für die $R_1$ ein Wasserstoffatom ist, mit Formol in Ameisensäure zur Reaktion bringt.

8. Verfahren zur Herstellung der Verbindungen gemäß Anspruch 1, für die A die $CH_2$-Gruppe und $R_1$ eine Alkylgruppe mit 2 bis 4 Kohlenstoffatomen ist, dadurch gekennzeichnet, daß man die entsprechenden Verbindungen gemäß Anspruch 1, für die A die $CH_2$-Gruppe und $R_1$ ein Wasserstoffatom ist, mit einem Säurechlorid der Formel

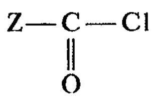

worin Z eine Alkylgruppe mit 1 bis 3 Kohlenstoffatomen ist, zur Reaktion bringt und dann die so erhaltene Verbindung mittels eines Hydrids reduziert.

9. Verfahren zur Herstellung der Verbindungen gemäß Anspruch 1 entsprechend der Formel (VII)

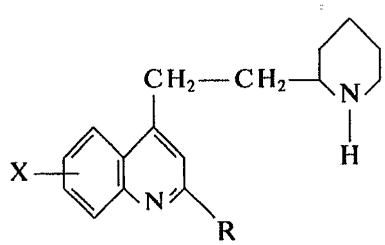

(VII)

dadurch gekennzeichnet, daß man die Pyridinderivate der Formel (VIII)

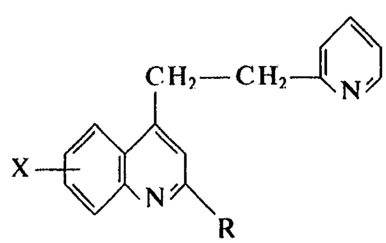

(VIII)

worin X und R dieselbe Bedeutung wie in Formel (I) von Anspruch 1 haben, katalytisch hydriert.

10. Verfahren zur Herstellung der Verbindungen gemäß Anspruch 1 entsprechend der Formel (XI)

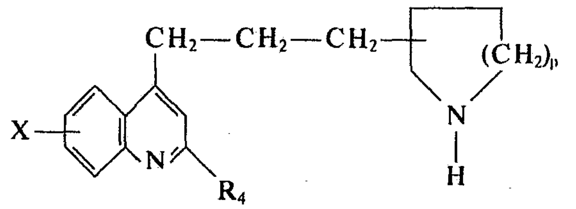

(XI)

worin X und p dieselbe Bedeutung wie in Formel (I) von Anspruch 1 haben und $R_4$ eine Phenylgruppe oder eine Phenylgruppe substituiert durch einen oder zwei Substituenten ausgewählt aus den Halogenatomen und $CF_3$ bedeutet, dadurch gekennzeichnet, daß man ein Metallderivat der Formel R″M, worin M ein Alkalimetall und R″ ein Wasserstoffatom, eine $NH_2$-Gruppe, eine disubstituierte Aminogruppe oder eine Alkyl- oder Arylgruppe bedeutet, mit einem Chinolinderivat der Formel (XII)

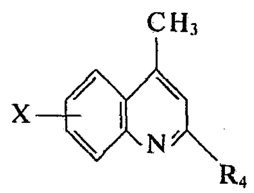

(XII)

worin X und $R_4$ dieselbe Bedeutung wie in Formel (XI) haben, zur Reaktion bringt, die so erhaltene Verbindung der Formel (XIII)

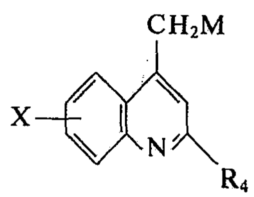

(XIII)

mit einer Verbindung der Formel (XIV)

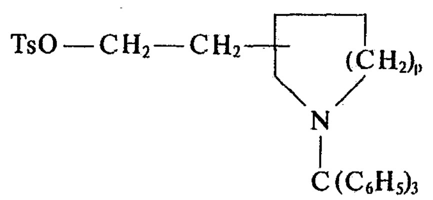

(XIV)

worin p dieselbe Bedeutung wie in Formel (XI) und Ts die Tosylgruppe bedeutet, zur Reaktion bringt und die so erhaltene Verbindung der Formel (XV)

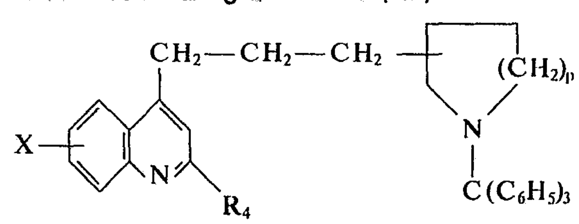

(XV)

der Einwirkung einer Säure unterwirft.

11. Arzneimittel zur Verwendung als Antiarrhythmikum, Hypnotikum, Antikonvulsivum und bei der Behandlung von Angstzuständen und verschiedenen psychoneurotischen Zuständen, dadurch gekennzeichnet, daß es als aktives Prinzip eine Verbindung entsprechend der Formel (I) von Anspruch 1 oder ein Salz einer solchen Verbindung mit einer pharmazeutisch annehmbaren Säure enthält.

12. Arzneimittel gemäß Anspruch 11, dadurch gekennzeichnet, daß es als aktives Prinzip die Verbindung der Formel

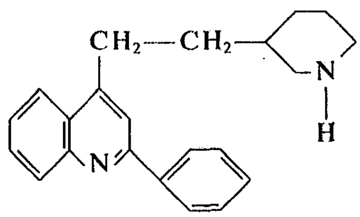

oder ein Salz dieser Verbindung mit einer pharmazeutisch annehmbaren Säure enthält.

**Claims**

1. Compounds of the general formula

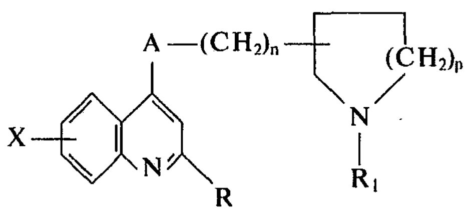

(I)

in which R represents a phenyl or pyridyl group, or a phenyl group which is substituted by one or two substituents selected from among halogen atoms (chlorine, fluorine, bromine) and the trifluoromethyl group, X is attached to the quinoline ring in position 6 and represents a hydrogen atom or a halogen atom (chlorine, fluorine or bromine) or a methoxy group, $R_1$ represents a hydrogen atom or an alkyl group containing 1 to 4 carbon atoms, A represents a CO or $CH_2$ group, n is an integer equal to 1 or 2, p is an integer equal to 1 or 2 and the

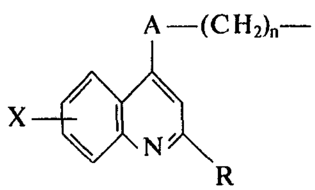

group is attached to the nitrogen-containing heterocyclic ring

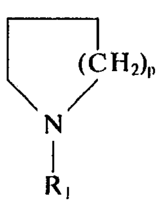

in the 2- or 3- position, their diastereoisomere, racemic forms and enantiomers, and their addition salts with inorganic or organic acids.

2. Compounds according to Claim 1, in which X is a hydrogen atom, R is a phenyl group and n is equal to 1.

3. Compound according to Claim 2, of the formula

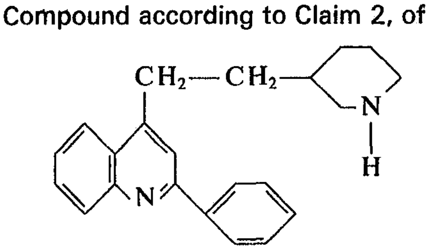

and its addition salts with inorganic or organic acids.

4. Process for the preparation of the compounds according to Claim 1, for which A is a $CH_2$ group, characterised in that the corresponding compounds according to Claim 1, for which A ist a CO group, are reduced.

5. Process for the preparation of the compounds according to Claim 1 for which A represents the CO group and $R_1$ a hydrogen atom, characterised in that a quinoline carboxylic acid ester of the formula

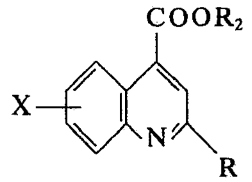

(II)

in which X and R have the same meanings as in formula (I) of Claim 1, and $R_2$ denotes a low molecular weight alkyl group, is condensed with an ester of the formula

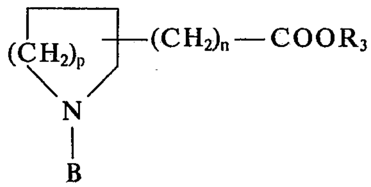

(III)

in which n and p have the same meaning as in formula (I) of Claim 1, $R_3$ denotes a low molecular weight alkyl group and B denotes a protective group from the amine group, and that the condensation product thus obtained is hydrolysed.

6. Process for the preparation of the compounds according to Claim 1 for which $R_1$ is an alkyl group, characterised in that the corresponding compounds according to Claim 1 for which $R_1$ is a hydrogen atom are reacted with an alkylating agent.

7. Process for the preparation of the compounds according to Claims 1 for which $R_1$ is a methyl group and A is a $CH_2$ group, characterised in that the corresponding compounds according to Claim 1 for which $R_1$ is a hydrogen atom are reacted with formaldehyde in formic acid.

8. Process for the preparation of the compounds according to Claim 1, for which A is the $CH_2$ group and $R_1$ is an alkyl group having 2 to 4 carbon atoms, characterised in that the corresponding compounds according to Claim 1 for which A is the $CH_2$ group and $R_1$ is a hydrogen atom are reacted with an acid chloride of the formula

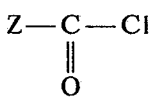

in which Z is an alkyl group having 1 to 3 carbon atoms, and the compound thus obtained is thereafter reduced by means of a hydride.

9. Process for the preparation of the compounds according to Claim 1 corresponding to the formula

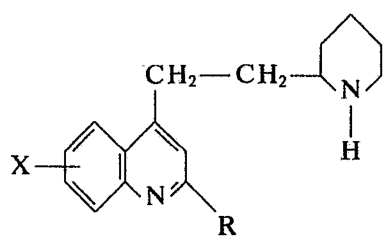

(VII)

characterised in that the pyridine derivatives of the formula

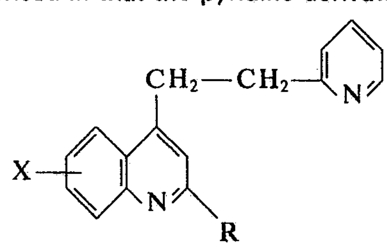

(VIII)

in which X and R have the same meaning as in formula (I) of Claim 1 are hydrogenated catalytically.

10. Process for the preparation of the compounds according to Claim 1 corresponding to the formula

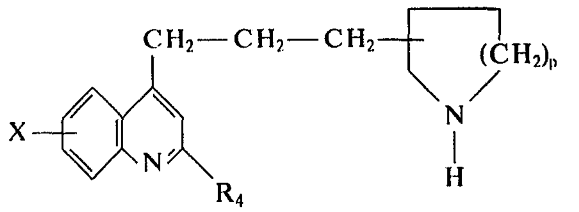

(XI)

in which X and p have the same meaning as in formula (I) of Claim 1 and $R_4$ denotes a phenyl group, or a phenyl group which is substituted by one or two substituents selected from among halogen atoms and $CF_3$, characterised in that a metal derivative of the formula R″M, in which M denotes an alkali metal and R″ denotes a hydrogen atom, an $NH_2$ group, a disubstituted amino group or an alkyl or aryl group, is reacted with a quinoline derivative of the formula

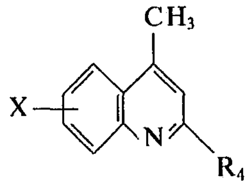

(XII)

in which X and $R_4$ have the same meaning as in formula (XI), the compound of the formula

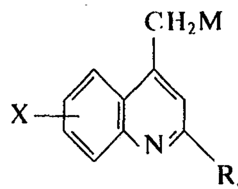

(XIII)

thus obtained is reacted with a compound of the formula

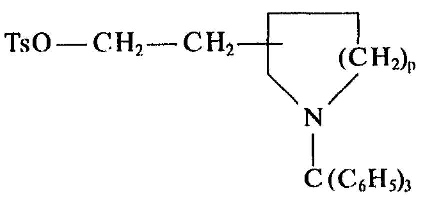

(XIV)

in which p has the same meaning as in formula (XI) and Ts denotes a tosyl group, and the compound of the formula

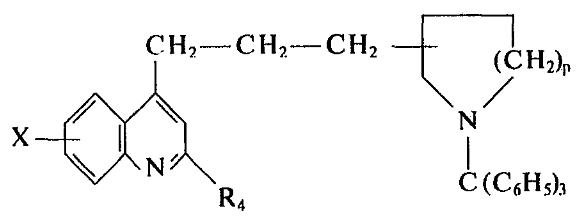

(XV)

thus obtained is treated with an acid.

11. Medicament for use as an antiarrhythmic, hypnotic or anticonvulsant agent and in the treatment of anxiety conditions and various psychoneurotic conditions, characterised in that it contains, as the active principle, a compound corresponding to formula (I) of Claim 1 or a salt of such a compound with a pharmaceutically acceptable acid.

12. Medicament according to Claim 11, characterised in that it contains, as the active principle, the compound of the formula

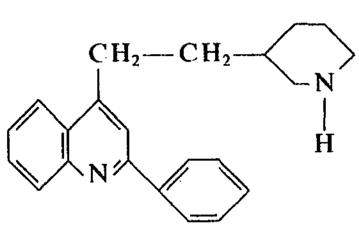

or a salt of this compound with a pharmaceutically acceptable acid.